# EUROPÄISCHE PATENTANMELDUNG

(11) **EP 3 088 878 A2**
(43) Veröffentlichungstag der Anmeldung: **02.11.2016**
(21) Anmeldenummer: 16167004.7
(22) Anmeldetag: 26.04.2016
(51) Int. Cl.: G01N 27/12, G01N 33/34

(54) **MESSVORRICHTUNG UND MESSVERFAHREN ZUR MESSUNG DER HARZTRÄNKUNG EINES SUBSTRATS**

(30) Priorität: 29.04.2015 AT 503482015
(71) Anmelder: Kompetenzzentrum Holz GmbH, 4040 Linz (AT)
(72) Erfinder: HOCHSTEINER, Sebastian, 9555 Glanegg (AT); LENZ, Simon, 9300 Sankt Veit an der Glan (AT); STULTSCHNIK, Jörg, 9125 Kühnsdorf (AT)
(74) Vertreter: Burger, Hannes

(57) **Zusammenfassung**

Verfahren zum Messen der Harztränkung eines Substrats (2) mit einer Messvorrichtung (1), welche drei voneinander beabstandete Elektroden (3, 4, 5) aufweist, welche ein zu prüfendes Substrat (2) berühren. Dabei wird eine Zeitmessung gestartet, wenn die elektrische Leitfähigkeit zwischen einer ersten und einer zweiten Elektrode (3, 4) oder ein davon abhängiger Parameter einen ersten Schwellwert erreicht, und beendet, wenn die elektrische Leitfähigkeit oder ein davon abhängiger Parameter zwischen der dritten Elektrode (5) und der ersten Elektrode (3) und/oder zwischen der dritten Elektrode (5) und der zweiten Elektrode (4) einen zweiten Schwellwert erreicht. Die genannte elektrische Leitfähigkeit wird dabei durch einen auf das Substrat (2) aufgebrachten Harztropfen (16) beziehungsweise durch das in das Substrat (2) einsickernde Harz beeinflusst. Darüber hinaus wird eine Vorrichtung (1) zur Durchführung des offenbarten Verfahrens angegeben.

## Beschreibung

Die Erfindung betrifft eine Messvorrichtung und Messverfahren zur Messung der Harztränkung eines Substrats, insbesondere von saugfähigem Papier.

Ein solches Messverfahren ist beispielsweise aus der holzverarbeitenden Industrie bekannt. Dort werden Substrate, insbesondere saugfähige Papiere, mit Harz getränkt, um dieses einerseits widerstandsfähiger zu machen, andererseits auch um sie mit einem Trägermaterial zu verbinden, beispielsweise mit einer Spanplatte oder Faserplatte. Um eine konstante Qualität beim Herstellungsprozess dieser Werkstoffe zu erhalten, ist es von großer Bedeutung, jene Zeit zu kennen, die das Substrat braucht, um mit flüssigem Harz getränkt zu werden.

Derzeit erfolgt diese Zeitmessung mit Hilfe eines Testsubstrats, das an zwei Seitenkanten aufgebogen und anschließend aus geringer Höhe mit waagrecht gehaltener Grundfläche in ein Harzbad fallengelassen wird. Dabei wird die Zeit vom Eintritts des Papiers in das Harzbad bis zu einer 80-prozentigen Flächenverfärbung der Papierprobe mittels Stoppuhr gemessen. Da die Flächenverfärbung mit freiem Auge erfasst wird, ist die Methode sehr personenspezifisch. Messergebnisse sind daher subjektiv und kaum reproduzierbar. Besonders problematisch ist dies, wenn helle, bedruckte Dekorpapiere vermessen werden.

Eine Aufgabe der Erfindung ist es daher, eine verbesserte Messvorrichtung und ein verbessertes Messverfahren zur Messung der Harztränkung eines Substrats anzugeben. Insbesondere sollen die Messergebnisse objektiv und gut reproduzierbar sein.

Die Aufgabe der Erfindung wird mit einer Messvorrichtung zur Messung der Harztränkung eines Substrats gelöst, welche
- drei voneinander beabstandete Elektroden umfasst,
- eine Leitfähigkeits-Messeinrichtung zur Messung der elektrischen Leitfähigkeit zwischen den Elektroden oder eines davon abhängigen Parameters,
- eine Zeitmesseinrichtung und
- eine mit der Leitfähigkeits-Messeinrichtung und der Zeitmesseinrichtung verbundene Steuereinheit, welche dazu eingerichtet ist, die Zeitmesseinrichtung zu starten, wenn ein erster Schwellwert der elektrischen Leitfähigkeit / des abhängigen Parameters zwischen einer ersten Elektrode und einer zweiten Elektrode erreicht wird, und die Zeitmesseinrichtung zu stoppen, wenn ein zweiter Schwellwert der elektrischen Leitfähigkeit / des abhängigen Parameters zwischen der dritten Elektrode und der ersten Elektrode und/oder zwischen der dritten Elektrode und der zweiten Elektrode erreicht wird.

Weiterhin wird die Aufgabe der Erfindung mit einem Verfahren zum Messen der Harztränkung eines Substrats mit einer Messvorrichtung gelöst, welche drei voneinander beabstandete Elektroden aufweist, welche ein zu prüfendes Substrat berühren, umfassend die Schritte
- Starten einer Zeitmessung, wenn die elektrische Leitfähigkeit zwischen einer ersten und einer zweiten Elektrode oder ein davon abhängiger Parameter einen ersten Schwellwert erreicht,
- Beenden der Zeitmessung, wenn die elektrische Leitfähigkeit oder ein davon abhängiger Parameter zwischen der dritten Elektrode und der ersten Elektrode und/oder zwischen der dritten Elektrode und der zweiten Elektrode einen zweiten Schwellwert erreicht.

Bei dem genannten Verfahren und der genannten Vorrichtung macht man sich den Umstand zu Nutze, dass das Harz elektrisch leitfähig ist. Wird das Harz (manuell oder maschinengesteuert) auf die Oberfläche des Substrats zwischen der ersten und der zweiten Elektrode appliziert, so wird zwischen diesen beiden Elektroden eine Erhöhung der elektrischen Leitfähigkeit respektive eine Abnahme des spezifischen elektrischen Widerstands festgestellt. Dieses Ereignis wird als Auslöser zum Starten einer Zeitmessung herangezogen. Nach und nach wird das Substrat nun mit dem Harz getränkt, das schließlich auch die dritte Elektrode erreicht. Dies führt zu einer Erhöhung der elektrischen Leitfähigkeit respektive eine Abnahme des spezifischen elektrischen Widerstands zwischen der dritten Elektrode und der ersten Elektrode und/oder zu einer Erhöhung der elektrischen Leitfähigkeit respektive eine Abnahme des spezifischen elektrischen Widerstands zwischen der dritten Elektrode und der zweiten Elektrode. Dieses Ereignis wird wiederum erfasst und zum Stoppen der Zeitmessung herangezogen. Die gemessene Zeitspanne dient nun als Maß für das Saugvermögen des Substrats für das jeweilige Harz. Selbstverständlich kann der Versuch beispielsweise bei verschiedenen Temperaturen, Luftfeuchtigkeiten, usw. durchgeführt werden. Wird der Versuch bei denselben Bedingungen durchgeführt, die auch beim Produktionsprozess vorliegen, so kann die ermittelte Zeit direkt für die Parametrierung des genannten Prozesses herangezogen werden. Vorteilhaft liefern die vorgestellte Messvorrichtung und das vorgestellte Messverfahren Messergebnisse, die objektiv und reproduzierbar sind.

Die Messvorrichtung und das Messverfahren eignen sich insbesondere für die Messung der Tränkung eines Substrats mit Harz. Neben Harz können jedoch generell auch andere leitfähige Flüssigkeiten herangezogen werden. Die Messvorrichtung und das Messverfahren eignen sich somit generell für die Messung der Tränkung eines Substrats mit einer elektrisch leitfähigen Flüssigkeit (Pentrationszeitmessung).

An dieser Stelle wird angemerkt, dass der Auslöser für das Starten und Stoppen der Messzeit nicht notwendigerweise eine Zunahme der elektrischen Leitfähigkeit im engeren Sinn (also Siemens pro Meter) ist, sondern auch davon abhängige Parameter für die Auslösung der Zeitmessung herangezogen werden können. Beispiele hierfür sind der spezifische elektrische Widerstand, welcher als Kehrwert der elektrischen Leitfähigkeit definiert ist, oder auch der ohmsche Widerstand oder der elektrische Leitwert zwischen den Elektroden. Das Harz führt zwischen den Elektroden zu einer Erhöhung der elektrischen Leitfähigkeit und des Leitwerts und zu einer Abnahme des spezifischen elektrischen Widerstands und des ohmschen Widerstands. Dementsprechend wird durch das Harz ein Schwellwert für die elektrische Leitfähigkeit oder den Leitwert überschritten, ein Schwellwert für den spezifischen elektrischen Widerstand und des ohmschen Widerstand dagegen unterschritten.

Vorteilhafte Ausgestaltungen und Weiterbildungen der Erfindung ergeben sich aus den Unteransprüchen sowie aus der Beschreibung in Zusammenschau mit den Figuren.

Günstig ist es, wenn die Messvorrichtung eine Zuführeinrichtung für das flüssige Harz aufweist, deren Austrittsöffnung zwischen der ersten Elektrode und der zweiten Elektrode angeordnet ist. Auf diese Weise kann das Harz reproduzierbar immer an derselben Stelle appliziert werden.

Vorteilhaft ist es in diesem Zusammenhang, wenn die Zuführeinrichtung durch eine Spritzenpumpe beziehungsweise einen Perfusor gebildet ist. Spritzenpumpen/Perfusoren sind insbesondere in der Medizintechnik bekannt und werden dort seit längerem erfolgreich eingesetzt. Durch die Übernahme beziehungsweise Verwendung dieser Mittel können die vorgestellte Messvorrichtung / das vorgestellte Messverfahren gut in die Praxis umgesetzt und zuverlässig betrieben / durchgeführt werden.

Von Vorteil ist es weiterhin, wenn vor dem Starten der Zeitmessung ein Harztropfen mit Hilfe einer Zuführvorrichtung maschinengesteuert auf der Oberfläche des Substrats zwischen die erste und die zweite Elektrode appliziert wird. Durch diese Maßnahme ist die Applikation des flüssigen Harzes objektiv und reproduzierbar.

Vorteilhaft ist es zudem, wenn die Messvorrichtung eine Steuerung aufweist, welche dazu eingerichtet ist, die Zuführeinrichtung im Hinblick auf eine Mindestgröße eines zwischen der ersten und der zweiten Elektrode befindlichen Harztropfens zu steuern oder zu regeln. Dementsprechend ist es bei dem vorgestellten Verfahren von Vorteil, wenn Harz über die Zuführvorrichtung nachgeführt wird und die Zuführeinrichtung im Hinblick auf eine Mindestgröße eines zwischen der ersten und der zweiten Elektrode befindlichen Harztropfens gesteuert oder geregelt wird. Auf diese Weise wird sichergestellt, dass das im Substrat einsickernde, flüssige Harz in ausreichender Menge nachgeliefert wird. Beispielsweise kann die Größe des Harztropfens optisch erfasst werden, etwa mit einer Kamera, oder es wird die elektrische Leitfähigkeit zwischen der ersten Elektrode und der zweiten Elektrode respektive ein davon abgeleiteter Parameter herangezogen. Insbesondere kann vorgesehen sein, dass die Messvorrichtung eine Steuerung aufweist, welche dazu eingerichtet ist, die Zuführeinrichtung im Hinblick auf eine Nachführung von flüssigem Harz anzusteuern, wenn die elektrische Leitfähigkeit zwischen der ersten Elektrode und der zweiten Elektrode oder ein davon abgeleiteter Parameter einen dritten Schwellwert erreicht. Dementsprechend ist es bei dem vorgestellten Verfahren von Vorteil, wenn flüssiges Harz über die Zuführvorrichtung nachgeführt wird, wenn die elektrische Leitfähigkeit zwischen der ersten Elektrode und der zweiten Elektrode oder ein davon abgeleiteter Parameter einen dritten Schwellwert erreicht. Konkret erfolgt eine Nachführung des Harzes, wenn ein Schwellwert für die elektrische Leitfähigkeit oder den Leitwert unterschritten wird oder ein Schwellwert für den spezifischen elektrischen Widerstand und des ohmschen Widerstand überschritten wird. Denkbar ist generell auch, eine Zuführeinrichtung empirisch einzustellen und die Größe des Harztropfens zu steuern.

Vorteilhaft ist es weiterhin, wenn die erste und die zweite Elektrode zungenförmig ausgebildet sind. Insbesondere sind diese an ihrem der dritten Elektrode zugewandten Ende aufgebogen und liegen in etwa linienförmig auf dem Substrat auf. Im Speziellen können die erste und die zweite Elektrode identisch geformt sein. Durch die zungenartige Form kann ein Substrat relativ leicht in der Messvorrichtung angebracht beziehungsweise in diese geklemmt werden.

Vorteilhaft ist es darüber hinaus, wenn die dritte Elektrode flächig ausgebildet ist, insbesondere eben mit einer Fläche von zumindest 100 cm². Dadurch kann ein ebenes Substrat ebenfalls vergleichsweise leicht in der Messvorrichtung angebracht werden.

Besonders vorteilhaft ist es zudem, wenn die erste und die zweite Elektrode auf einer ersten Seite des Substrats angeordnet werden, und die dritte Elektrode auf einer der ersten Seite gegenüberliegenden, zweiten Seite des Substrats angeordnet wird. Dadurch hat die Größe des Harztropfens keinen oder nur geringen Einfluss auf Zeit, die das Harz zum Erreichen der dritten Elektrode benötigt. Insbesondere ist das Substrat bei dieser Variante als Blatt beziehungsweise als Plättchen ausgebildet, das heißt als ebener Körper mit vergleichsweise geringer Dicke.

Vorteilhaft ist es schließlich auch, wenn alle Elektroden auf einer ersten Seite des Substrats angeordnet werden. Durch Wählen eines Abstands der dritten Elektrode zur ersten/zweiten Elektrode kann im Wesentlichen auch der Zeitbereich eingestellt werden, den das Harz zum Erreichen der dritten Elektrode benötigt. Größere Abstände verlängern die genannte Zeit, kleinere Abstände verringern sie. Insbesondere bei sehr dünnen Substraten und/oder sehr dünnflüssigen Harzen kann die Messzeit auf diese Weise in einen leicht handhabbaren Bereich gebracht werden.

Zum besseren Verständnis der Erfindung wird diese anhand der nachfolgenden Figuren näher erläutert.

Es zeigen jeweils in stark vereinfachter, schematischer Darstellung:
- Fig. 1: ein erstes Beispiel einer Messvorrichtung mit zwei zungenförmigen Elektroden und einer gegenüberliegend angeordneten plattenförmigen Elektrode und
- Fig. 2: drei stiftförmige Elektroden, welche ein Substrat auf einer Seite berühren.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und bei einer Lageänderung sinngemäß auf die neue Lage zu übertragen.

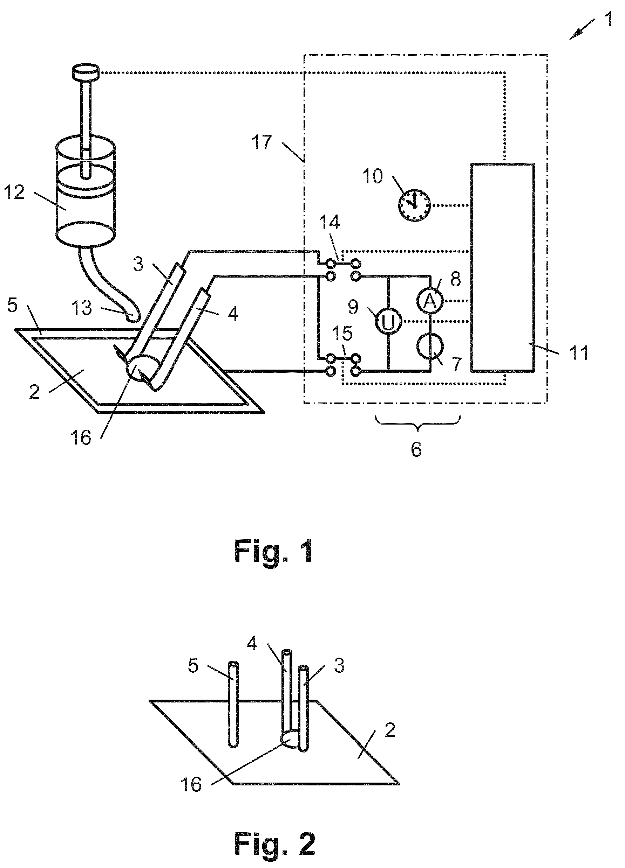

Fig. 1 zeigt eine Messvorrichtung 1 zur Messung der Harztränkung eines Substrats 2, welche drei voneinander beabstandete Elektroden 3, 4 und 5 umfasst. In diesem konkreten Beispiel sind die erste Elektrode 3 und die zweite Elektrode 4 zungenförmig ausgebildet. Vorteilhaft sind diese, wie dargestellt, an ihrem der dritten Elektrode 5 zugewandten Ende aufgebogen und liegen in etwa linienförmig auf dem Substrat 2 auf. Die dritte Elektrode 5 ist in diesem Beispiel dagegen flächig und eben ausgebildet und weist insbesondere eine Fläche von zumindest 100 cm² auf. Prinzipiell können die Elektroden 3..5 aber auch anders geformt sein (vergleiche Fig. 2).

Die Elektroden 3..5 sind in diesem Beispiel aus Metall gefertigt. Konkret bestehen die erste und die zweite Elektrode 3 und 4 aus Stahl, und die dritte Elektrode 5 besteht aus Aluminium. Selbstverständlich können aber auch andere Elektrodenmaterialien eingesetzt werden, insbesondere Kupfer, Messing oder Bronze.

Die Messvorrichtung 1 weist weiterhin eine Leitfähigkeits-Messeinrichtung 6 zur Messung der elektrischen Leitfähigkeit zwischen den Elektroden 3..5 oder eines von der genannten elektrischen Leitfähigkeit abhängigen Parameters auf. Die Leitfähigkeits-Messeinrichtung 6 umfasst in diesem Beispiel konkret eine Spannungsquelle 7, ein Strommessgerät 8 sowie ein Spannungsmessgerät 9.

Darüber hinaus umfasst die Messvorrichtung 1 eine Zeitmesseinrichtung 10, sowie eine mit der Leitfähigkeits-Messeinrichtung 6 und der Zeitmesseinrichtung 10 verbundene Steuereinheit 11.

Die Steuereinheit 11 ist dazu eingerichtet, die Zeitmesseinrichtung 10 zu starten, wenn ein erster Schwellwert der elektrischen Leitfähigkeit / des abhängigen Parameters zwischen einer ersten Elektrode 3 und einer zweiten Elektrode 4 erreicht wird, und die Zeitmesseinrichtung 10 zu stoppen, wenn ein zweiter Schwellwert der elektrischen Leitfähigkeit / des abhängigen Parameters zwischen der dritten Elektrode 5 und der ersten Elektrode 3 und/oder zwischen der dritten Elektrode 5 und der zweiten Elektrode 4 erreicht wird beziehungsweise ein solches Ereignis durch die Steuereinheit 11 festgestellt oder detektiert wird. Beispielsweise kann die Steuereinheit 11 in Form eines Mikroprozessors, eines Computers oder in Form einer Speicherprogrammierbaren Steuerung (SPS) ausgebildet sein.

Die Messvorrichtung 1 weist schließlich eine optionale Zuführeinrichtung 12 für das flüssige Harz auf, deren Austrittsöffnung 13 zwischen der ersten Elektrode 3 und der zweiten Elektrode 4 angeordnet ist. Die Zuführeinrichtung 12 kann, wie in Fig. 1 dargestellt, insbesondere durch eine Spritzenpumpe beziehungsweise einen Perfusor gebildet sein. Zudem umfasst die Messvorrichtung 1 optionale Schalter 14 und 15.

Die Funktion der in der Fig. 1 dargestellten Messvorrichtung 1 ist nun wie folgt:
In einem ersten Schritt wird das Substrat 2 (z.B. saugfähiges Papier) zwischen die Elektroden 3..5 eingelegt beziehungsweise eingespannt. Dass Substrat 2 wird daher auf der Unterseite von der dritten Elektrode 5 berührt, auf der gegenüberliegenden Oberseite von der ersten und der zweiten Elektrode 3, 4. Die erste und die zweite Elektrode 3, 4 können insbesondere federnd ausgebildet sein, sodass das Substrat 2 mit einer definierten Kontaktraft belastet wird.

Mit Hilfe der Zuführvorrichtung 12 wird nun ein Harztropfen 16 maschinengesteuert auf der Oberfläche des Substrats 2 zwischen die erste und die zweite Elektrode 3, 4 appliziert. Dadurch ist das Applizieren objektiv und reproduzierbar. Selbstverständlich könnte das flüssige Harz auch manuell appliziert werden.

In einem Initialzustand befinden sich die Schalter 14 und 15 in der in der Fig. 1 gezeigten Stellung. Mit Hilfe der Spannungsquelle 7 wird demzufolge ein über das Strommessgerät 8, die erste Elektrode 3, das Substrat 2 beziehungsweise den Harztropfen 16, sowie die zweite Elektrode 4 fließender elektrischer Strom erzeugt. Mit Hilfe des Spannungsmessgeräts (Voltmeters) 9 und des Strommessgeräts (Amperemeters) 8 kann nun der ohmsche Widerstand respektive der Leitwert ermittelt werden. Die metallischen Anteile im Stromkreis, wie zum Beispiel die erste und zweite Elektrode 3, 4 sowie Drähte, Leiterbahnen und dergleichen können im Prinzip vernachlässigt werden, da deren Leitwert um ein Vielfaches über dem Leitwert des Harztropfens 16 / des Substrats 2 liegt. Selbstverständlich können diese Anteile aber auch berücksichtigt werden. Zur Ermittlung des betreffenden Leitwerts können die erste und die zweite Elektrode 3, 4 kurzgeschlossen werden. Ist der Abstand zwischen der ersten und zweiten Elektrode 3, 4 bekannt, so kann aus kann aus den Messwerten für Strom und Spannung auch die elektrische Leitfähigkeit und der spezifische elektrische Widerstand ermittelt werden.

Überschreitet die ermittelte elektrische Leitfähigkeit (oder auch der Leitwert) einen ersten Schwellwert, dann wird eine Zeitmessung gestartet. Die Zeitmessung kann in analoger Weise auch gestartet werden, wenn der spezifische elektrische Widerstand oder der ohmsche Widerstand einen ersten Schwellwert unterschreitet.

Das flüssige Harz sickert nun in das Substrat 2 ein und erreicht irgendwann die dritte Elektrode 5. Die Zeitmessung wird beendet, wenn die elektrische Leitfähigkeit oder ein davon abhängiger Parameter zwischen der dritten Elektrode 5 und der ersten Elektrode 3 und/oder zwischen der dritten Elektrode 5 und der zweiten Elektrode 4 einen zweiten Schwellwert erreicht. Die gemessene Zeitspanne kann über eine Anzeigeeinheit (nicht dargestellt) angezeigt oder auch automatisiert weiterverarbeitet werden, beispielsweise in einem Computer.

Damit stets ausreichend Harz zur Verfügung steht, kann vorgesehen sein, dass Harz über die Zuführvorrichtung 12 laufend nachgeführt wird und die Zuführeinrichtung 12 im Hinblick auf eine Mindestgröße des zwischen der ersten und der zweiten Elektrode 3, 4 befindlichen Harztropfens 16 gesteuert oder geregelt wird. Beispielsweise kann die Größe des Harztropfens 16 optisch durch eine nicht dargestellte Kamera erfasst werden. Denkbar wäre auch, dass die Zuführeinrichtung 12 im Hinblick auf eine Nachführung von flüssigem Harz angesteuert wird, wenn die elektrische Leitfähigkeit zwischen der ersten Elektrode 3 und der zweiten Elektrode 4 oder ein davon abgeleiteter Parameter einen dritten Schwellwert erreicht. Bei dieser Variante wird also Harz nachgeführt, wenn die elektrische Leitfähigkeit / der elektrische Leitwert zwischen der ersten Elektrode 3 und der zweiten Elektrode 4 den dritten Schwellwert unterschreitet oder der spezifische elektrische Widerstand / der ohmsche Widerstand zwischen der ersten Elektrode 3 und der zweiten Elektrode 4 den dritten Schwellwert überschreitet. Denkbar ist natürlich auch, die Zuführeinrichtung 12 empirisch einzustellen und die Größe des Harztropfens 16 respektive die Menge an Harz pro Zeiteinheit zu steuern. Die Steuerung/Regelung der Zuführeinrichtung 12 kann generell von der Steuerung 11 oder einer anderen (beispielsweise in der Zuführeinrichtung 12 eingebauten) Steuerung übernommen werden.

In dem in Fig. 1 gezeigten Beispiel ist eine (einzige) Leitfähigkeits-Messeinrichtung 6 vorgesehen, welche mit Hilfe der Schalter 14, 15 in unterschiedlicher Weise an die Elektroden 3..5 geschaltet werden kann, um die elektrische Leitfähigkeit zwischen der ersten Elektrode 3 und der zweiten Elektrode 4 (Schalter 14 obere Stellung, Schalter 15 obere Stellung), zwischen der ersten Elektrode 3 und der dritten Elektrode 5 (Schalter 14 obere Stellung, Schalter 15 untere Stellung) oder zwischen der zweiten Elektrode 4 und der dritten Elektrode 5 (Schalter 14 untere Stellung, Schalter 15 untere Stellung) ermitteln zu können. Insbesondere kann vorgesehen sein, dass die Schalter 14, 15 in periodischen Zeitabständen umgeschaltet werden, um die oben erwähnten drei Messzustände zu erhalten. Auf diese Weise kann mit derselben Leitfähigkeits-Messeinrichtung 6 einerseits die Zeitmesseinrichtung 10 gestartet und die Zuführeinrichtung 12 gesteuert werden, andererseits kann damit auch die Zeitmessung wieder gestoppt werden. Letzteres kann basierend auf der elektrischen Leitfähigkeit zwischen der dritten Elektrode 5 und der ersten Elektrode 3 oder basierend auf der elektrischen Leitfähigkeit zwischen der dritten Elektrode 5 und der zweiten Elektrode 4 erfolgen. Insbesondere kann die Zeitmessung gestoppt werden, wenn eine der beiden Messwerte einen zweiten Schwellwert erreicht. Denkbar wäre aber auch, dass beide Messwerte einen zweiten Schwellwert erreichen müssen, damit die Zeitmessung gestoppt wird.

Die Schalter 14, 15 sind zwar vorteilhaft aber nicht zwingend für die Erfindung. Denkbar wäre auch, dass mehrere Leitfähigkeits-Messeinrichtungen 6 vorgesehen sind, wodurch die Schalter 14 und 15 teilweise oder ganz entfallen können. Denkbar wäre auch, dass anstelle der Spannungsquelle 7 eine Stromquelle verwendet wird.

An dieser Stelle wird angemerkt, dass die in der Fig. 1 dargestellte funktionale Segmentierung der Messvorrichtung 1 in der Realität nicht zwingend vorliegen muss und in der Fig. 1 vorwiegend funktionale Blöcke dargestellt sind, die in verschiedener Ausprägung in real vorliegenden physikalischen Baueinheiten integriert sein können. So ist zum Beispiel eine Zeitmesseinrichtung 10 häufig eine Standardfunktion von Steuereinheiten 11 (Mikrocontroller, Computer, SPS) und muss daher nicht extra vorgesehen sein. Dasselbe gilt für die Stromquelle 7, die Strommesseinrichtung 8 und die Spannungsmesseinrichtung 9, die ebenfalls integraler Bestandteil der Steuerung 11 sein können.

Insbesondere sind die Steuerung 11, die Leitfähigkeits-Messeinrichtung 6, die Zeitmesseinrichtung 10 sowie die Schalter 14 und 15 in einem gemeinsamen Gehäuse 17 untergebracht, und die Elektroden 3..5 und die Zuführeinrichtung 12 sind extern daran angeschlossene Baueinheiten. Selbstverständlich ist auch diese Segmentierung nicht zwingend, und die genannten Einrichtungen können auch in anderer Art und Weise in Gehäusen integriert sein. An dieser Stelle wird auch angemerkt, dass der Perfusor 12 nicht zwingend vertikal ausgerichtet ist, sondern natürlich auch horizontal ausgerichtet sein kann.

Fig. 2 zeigt nun eine weitere Möglichkeit, wie die Elektroden 3..5 geformt und angeordnet sein können. Konkret sind hier alle drei Elektroden 3..5 stiftförmig ausgebildet und an der dem Substrat 2 zugewandten Seite gerundet. Zudem sind alle drei Elektroden 3..5 auf einer Seite des Substrats 2 angeordnet. Der Abstand zwischen der ersten Elektrode 3 und die zweiten Elektrode 4 ist dabei vorzugsweise etwas geringer als der Abstand der dritten Elektrode 5 zur ersten Elektrode 3 / zweiten Elektrode 4, so wie dies in der Fig. 2 dargestellt ist. Selbstverständlich kann die in der Fig. 2 gezeigte Elektrodenform auch für die erste und zweite Elektrode 3, 4 der in der Fig. 1 dargestellten Messvorrichtung 1 verwendet werden. Umgekehrt kann auch die in der Fig. 1 für die erste und zweite Elektrode 3, 4 verwendete Elektrodenform für die Elektroden 3..5 der Fig. 2 verwendet werden. Mischformen sind ebenfalls denkbar.

Die Ausführungsbeispiele zeigen mögliche Ausführungsvarianten einer erfindungsgemäßen Messvorrichtung 1, wobei an dieser Stelle bemerkt sei, dass die Erfindung nicht auf die speziell dargestellten Ausführungsvarianten desselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mit umfasst.

Insbesondere wird festgehalten, dass die dargestellten Vorrichtungen in der Realität auch mehr oder auch weniger Bestandteile als dargestellt umfassen können.

Der Ordnung halber sei abschließend darauf hingewiesen, dass zum besseren Verständnis des Aufbaus der Messvorrichtung 1 diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

### Bezugszeichenaufstellung

- 1: Messvorrichtung
- 2: Substrat (saugfähiges Papier)
- 3: erste Elektrode
- 4: zweite Elektrode
- 5: dritte Elektrode
- 6: Leitfähigkeits-Messeinrichtung
- 7: Spannungsquelle
- 8: Strommessgerät (Amperemeter)
- 9: Spannungsmessgerät (Voltmeter)
- 10: Zeitmesseinrichtung (Stoppuhr)
- 11: Steuerung (Mikrokontroller, Computer, SPS)
- 12: Zuführeinrichtung (Perfusor)
- 13: Austrittsöffnung
- 14: Schalter
- 15: Schalter
- 16: Harztropfen
- 17: Gehäuse

## Patentansprüche

1. Messvorrichtung (1) zur Messung der Harztränkung eines Substrats (2), umfassend
- drei voneinander beabstandete Elektroden (3, 4, 5),
- eine Leitfähigkeits-Messeinrichtung (6) zur Messung der elektrischen Leitfähigkeit zwischen den Elektroden (3, 4, 5) oder eines davon abhängigen Parameters,
- eine Zeitmesseinrichtung (10) und
- eine mit der Leitfähigkeits-Messeinrichtung (6) und der Zeitmesseinrichtung (10) verbundene Steuereinheit (11), welche dazu eingerichtet ist, die Zeitmesseinrichtung (10) zu starten, wenn ein erster Schwellwert der elektrischen Leitfähigkeit / des abhängigen Parameters zwischen einer ersten Elektrode (3) und einer zweiten Elektrode (4) erreicht wird, und die Zeitmesseinrichtung (10) zu stoppen, wenn ein zweiter Schwellwert der elektrischen Leitfähigkeit / des abhängigen Parameters zwischen der dritten Elektrode (5) und der ersten Elektrode (3) und/oder zwischen der dritten Elektrode (5) und der zweiten Elektrode (4) erreicht wird.

2. Messvorrichtung (1) nach Anspruch 1, **gekennzeichnet durch** eine Zuführeinrichtung (12) für das flüssige Harz, deren Austrittsöffnung (13) zwischen der ersten Elektrode (3) und der zweiten Elektrode (4) angeordnet ist.

3. Messvorrichtung (1) nach Anspruch 2, **dadurch gekennzeichnet, dass** die Zuführeinrichtung (12) durch eine Spritzenpumpe beziehungsweise einen Perfusor gebildet ist.

4. Messvorrichtung (1) nach einem der Ansprüche 2 bis 3, **gekennzeichnet durch** eine Steuerung (11), welche dazu eingerichtet ist, die Zuführeinrichtung (12) im Hinblick auf eine Mindestgröße eines zwischen der ersten und der zweiten Elektrode (3, 4) befindlichen Harztropfens (16) zu steuern oder zu regeln.

5. Messvorrichtung (1) nach einem der Ansprüche 2 bis 3, **gekennzeichnet durch** eine Steuerung (11), welche dazu eingerichtet ist, die Zuführeinrichtung (12) im Hinblick auf eine Nachführung von flüssigem Harz anzusteuern, wenn die elektrische Leitfähigkeit zwischen der ersten Elektrode (3) und der zweiten Elektrode (4) oder ein davon abgeleiteter Parameter einen dritten Schwellwert erreicht.

6. Messvorrichtung (1) nach einem der Ansprüche 1 bis 5, **dadurch gekennzeichnet, dass** die erste und die zweite Elektrode (3, 4) zungenförmig ausgebildet sind.

7. Messvorrichtung (1) nach einem der Ansprüche 1 bis 6, **dadurch gekennzeichnet, dass** die dritte Elektrode (5) flächig ausgebildet ist.

8. Verfahren zum Messen der Harztränkung eines Substrats (2) mit einer Messvorrichtung (1), welche drei voneinander beabstandete Elektroden (3, 4, 5) aufweist, welche ein zu prüfendes Substrat (2) berühren, umfassend die Schritte
- Starten einer Zeitmessung, wenn die elektrische Leitfähigkeit zwischen einer ersten und einer zweiten Elektrode (3, 4) oder ein davon abhängiger Parameter einen ersten Schwellwert erreicht,
- Beenden der Zeitmessung, wenn die elektrische Leitfähigkeit oder ein davon abhängiger Parameter zwischen der dritten Elektrode (5) und der ersten Elektrode (3) und/oder zwischen der dritten Elektrode (5) und der zweiten Elektrode (4) einen zweiten Schwellwert erreicht.

9. Verfahren nach Anspruch 8, **dadurch gekennzeichnet, dass** vor dem Starten der Zeitmessung ein Harztropfen (16) mit Hilfe einer Zuführvorrichtung (12) maschinengesteuert auf der Oberfläche des Substrats (2) zwischen die erste und die zweite Elektrode (3, 4) appliziert wird.

10. Verfahren nach Anspruch 9, **dadurch gekennzeichnet, dass** flüssiges Harz über die Zuführvorrichtung (12) nachgeführt wird und die Zuführeinrichtung (12) im Hinblick auf eine Mindestgröße eines zwischen der ersten und der zweiten Elektrode (3, 4) befindlichen Harztropfens (16) gesteuert oder geregelt wird.

11. Verfahren nach Anspruch 9, **dadurch gekennzeichnet, dass** flüssiges Harz über die Zuführvorrichtung (12) nachgeführt wird, wenn die elektrische Leitfähigkeit zwischen der ersten Elektrode (3) und der zweiten Elektrode (4) oder ein davon abgeleiteter Parameter einen dritten Schwellwert erreicht.

12. Verfahren nach einem der Ansprüche 8 bis 11, **dadurch gekennzeichnet, dass** als Substrat (2) saugfähiges Papier verwendet wird.

13. Verfahren nach einem der Ansprüche 8 bis 12, **dadurch gekennzeichnet, dass** die erste und die zweite Elektrode (3, 4) auf einer ersten Seite des Substrats (2) angeordnet werden, und dass die dritte Elektrode (5) auf einer der ersten Seite gegenüberliegenden, zweiten Seite des Substrats (2) angeordnet wird.

14. Verfahren nach einem der Ansprüche 8 bis 12, **dadurch gekennzeichnet, dass** alle Elektroden (3..5) auf einer ersten Seite des Substrats (2) angeordnet werden.
